(19) Europäisches Patentamt
European Patent Office
Office européen des brevets

(11) **EP 2 323 547 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**15.12.2021 Patentblatt 2021/50**

(45) Hinweis auf die Patenterteilung:
**10.10.2012 Patentblatt 2012/41**

(21) Anmeldenummer: **09757301.8**

(22) Anmeldetag: **04.06.2009**

(51) Int Cl.:
***A61M 1/36*** *(2006.01)* ***A61B 5/029*** *(2006.01)*

(86) Internationale Anmeldenummer:
**PCT/EP2009/004019**

(87) Internationale Veröffentlichungsnummer:
**WO 2009/146918 (10.12.2009 Gazette 2009/50)**

(54) **VORRICHTUNG ZUR BESTIMMUNG DES BLUTVOLUMENS UND/ODER BLUTVOLUMENSTROMS UND VERFAHREN ZUM BETREIBEN DERSELBEN**

DEVICE FOR DETERMINING THE BLOOD VOLUME AND/OR BLOOD VOLUMETRIC FLOW AND METHOD FOR OPERATING THE SAME

DISPOSITIF POUR DETERMINER LE VOLUME SANGUIN ET/OU LE DEBIT SANGUIN ET PROCEDE DE FONCTIONNEMENT ASSOCIE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **04.06.2008 DE 102008026708**

(43) Veröffentlichungstag der Anmeldung:
**25.05.2011 Patentblatt 2011/21**

(73) Patentinhaber: **Edwards Lifesciences IPRM AG**
**1260 Nyon (CH)**

(72) Erfinder:
• **PFEIFFER, Ulrich**
**81667 München (DE)**

• **KNOLL, Reinhold**
**81543 München (DE)**

(74) Vertreter: **Eisenführ Speiser**
**Patentanwälte Rechtsanwälte PartGmbB**
**Postfach 31 02 60**
**80102 München (DE)**

(56) Entgegenhaltungen:
| | |
|---|---|
| **WO-A1-02/053212** | **DE-A1- 4 214 068** |
| **US-A- 3 888 239** | **US-A- 5 217 019** |
| **US-A1- 2005 284 815** | **US-A1- 2006 211 947** |
| **US-A1- 2007 175 828** | |

EP 2 323 547 B2

## Beschreibung

Die Erfindung betrifft eine Vorrichtung zur Bestimmung des Blutvolumens und/oder Blutvolumenstroms eines Kreislaufabschnitts, mit einem Flüssigkeitsspeicher zur Bevorratung einer Messflüssigkeit, einem Injektionsmittel, insbesondere einem Katheter (11)oder einer Kanüle, zur Injektion der Messflüssigkeit in den Blutkreislauf, mindestens einer zwischen dem Flüssigkeitsspeicher und dem Injektionsmittel angeordneten ersten Pumpeinrichtung, Mitteln zur Bestimmung mindestens einer Eigenschaft der Messflüssigkeit vor der Injektion in den Blutkreislauf, weiteren Mitteln zur Bestimmung der betreffenden Eigenschaft im Blutkreislauf stromab der Injektionsstelle sowie einer Auswerteeinrichtung zur Berechnung von Blutvolumen oder Blutvolumenstrom in Abhängigkeit von den bestimmten Eigenschaften. Die Erfindung betrifft ferner eine derartige Vorrichtung mit Mitteln zur Konditionierung der Messflüssigkeit sowie ein Verfahren zum Betreiben derartiger Vorrichtungen.

## Stand der Technik

Vorrichtungen der eingangs genannten Art sind aus der Offenlegungsschrift DE 42 14 068 A1 bekannt. Die dort beschriebenen Vorrichtungen bestehen aus einem Flüssigkeitsvorrat in Form eines Infusionsbeutels, welcher mit einer gekühlten Messflüssigkeit befüllt ist. Eine geeignete Messflüssigkeit kann beispielsweise eine isotone Elektrolytlösung (zum Beispiel Kochsalzlösung, Ringerlösung, Lithiumsalzlösung), eine isotone nichtionische Lösung (z.B. Glukoselösung) oder fettfreie Nährlösung, eine Farbstofflösung (Evans Blau, Indocyaningrün), aber auch eine hypertone Kochsalzlösung sein. Die Messflüssigkeit wird über eine Tropfkammer mittels einer Pumpeinrichtung durch eine Ventileinrichtung einem Katheter zugeführt, mittels dessen die Messflüssigkeit pulsierend in den Blutkreislauf des Patienten injiziert wird. Der Zustrom der im Vergleich zur Körpertemperatur kühleren Messflüssigkeit beeinflusst die Bluttemperatur stromab der Injektionsstelle.

Dieses als Thermodilution bezeichnete Verfahren wird beispielsweise zur Bestimmung des Herzzeitvolumens angewendet. Dabei wird ein mit mehreren Kanälen ausgestatteter, beispielsweise aus der Patentschrift US 4,817,624 bekannter Katheter (z. B. Pulmonalis-Thermodilution-Einschwemmkatheter) derart im Blutkreislauf platziert, dass über einen ersten Kanal vor der rechten Herzkammer die gekühlte Messflüssigkeit injiziert und mittels eines an einem anderen Kanal angeordneten Temperaturfühler die Temperatur des Blutstroms hinter der rechten Herzkammer bestimmt wird. Von einer Auswerteeinrichtung kann nachfolgend aus dem Volumen der zugeführten Messflüssigkeit, den Temperaturen des zugeführten Injektats und des Blutes sowie dem Temperaturverlauf im Blutstrom stromab der Injektionsstelle mit Hilfe der Stewart-Hamilton-Formel das Herzzeitvolumen HZV berechnet werden.

Dabei gilt:

$$HZV = \frac{V_I * (T_B - T_I) * K}{\int (\Delta T * t) dt}$$

mit

$V_I$ =    Injektatvolumen
$T_B$ =    Bluttemperatur
$T_I$ =    Injektattemperatur
$K$ =    Korrekturfaktor für spezifisches Gewicht und spezifische Wärme von Injektat und Blut
$t$ =    Zeit

Zusätzlich lassen sich mit aus der Praxis bekannten Methoden aus Herzzeitvolumen und beispielsweise mittlerer Durchgangszeit, medianer Transitzeit, Verhältnis der Steilheiten des ansteigenden und abfallenden Teils der Indikatorverdünnungskurve, das durchlaufene Volumen und Teilvolumina daraus bestimmen. Bei einer zentralvenöser Indikatorinjektion und arterieller Indikatormessung sind dieses zum Beispiel das intrathorakale Blutvolumen, das globale end-diastolische Volumen, das tatsächliche kardiale Füllungsvolumen und das extravasale Lungenwasser.

Eine intermittierend durchgeführte Indikatorverdünnung kann dazu verwendet werden, eine andere, kontinuierlich durchführbare Methode der Fluss- und gegebenenfalls Volumenbestimmung wiederholt zu kalibrieren. Häufig ist dieses Vorgehen die Kalibrierung der arteriellen Pulskonturmethode zur kontinuierlichen Herzzeitvolumenbestimmung. Es kann aber auch bei anderen kontinuierlichen HZV-Bestimmungsmethoden Anwendung finden, beispielsweise bei der transösophagealen oder der transthorakalen Echokardiographie, der ösophagealen oder externen Ultraschall-Dopplersonografie oder der elektrischen thorakalen Bioimpedanzmethode. Allen kontinuierlichen Methoden ist dabei in der Regel gemein, dass sie per se nicht sehr genaue Messergebnisse liefern, jedoch durch häufigere Rekalibrierung in eine wesentlich höhere Genauigkeitsklasse gehoben werden.

Die Thermodilution kann auch transpulmonal gemessen werden. Alternativ zur Stewart Hamilton Formel kann die Auswertung mit Hilfe der Kreuzkorrelation nach Yelderman erfolgen wie in US 4,507,974.

Nach einem ersten in DE 42 14 068 A1 offenbarten Ausführungsbeispiel besteht die Pumpeinrichtung aus einer Schlauchpumpe, bei welcher die vom Flüssigkeitsvorrat zum Katheter führende Schlauchleitung von einer Nockenanordnung stromab aufeinanderfolgend komprimiert wird. Dabei wird die in der Schlauchleitung befindliche Flüssigkeitssäule in Richtung der Injektionsstelle verschoben und passiert ein stromab der Pumpeinrichtung angeordnetes Rückschlagventil, welches nur bei einer Strömung vom Flüssigkeitsvorrat zum Katheter öffnet und ein Rückfließen unterbindet.

Nach einem anderen Ausführungsbeispiel in DE 42 14 068 A1 ist die Pumpeinrichtung als mit einem Kolbenantrieb ausgestattete Zylinder-/Kolbenanordnung ausgeführt, welche über ein Zwei-Wege-Ventil mit dem Flüssigkeitsvorrat sowie dem Katheter strömungsverbindbar ist. In einer ersten Ventilstellung fließt die Messflüssigkeit aus dem Flüssigkeitsvorrat in die Zylinder-/Kolbenanordnung, während der Kolben von der Antriebseinrichtung bei sich vergrößerndem Kammervolumen zurückgezogen wird. Nach dem Umschalten des Ventils kann die in der Zylinder-/Kolbenanordnung gespeicherte Messflüssigkeit dem Katheter zugeführt werden, wobei der Kolben unter Verkleinerung des Kammervolumens nach vorne fährt.

Hinsichtlich der Durchführung der Thermodilution und des Einsatzes gattungsgemäßer Vorrichtungen zur Bestimmung des Blutvolumens oder -volumenstroms eines Kreislaufabschnitts wird ausdrücklich auf die Beschreibung in der Offenlegungsschrift DE 42 14 068 A1 verwiesen.

Zur Vermeidung von Embolien ist in beiden Ausführungsbeispielen stromab der Pumpeinrichtung ein Luftsensor in der zum Katheter führenden Schlauchleitung angeordnet, welcher mit der Steuerung der Thermodilutionsvorrichtung verbunden ist. Der Infusionsbeutel kann zwar durch geeignete Mittels komprimiert werden, um durch den dabei erzeugten Überdruck ein Eindringen von Luft zu verhindert. Dennoch kann nicht mit Sicherheit ausgeschlossen werden, dass sich im Injektat Luftblasen bilden, so dass der Injektionsvorgang unterbrochen werden muss, falls diese vom Luftsensor ermittelt werden. Darüber hinaus besteht die Möglichkeit, dass sich das Injektat auf dem Weg vom Flüssigkeitsvorrat zum Katheter, insbesondere während des Verweilens in der Zylinder-/Kolbenanordnung, erwärmt und damit das Messergebnis beeinflusst.

Patentschrift US 5,037,396 offenbart eine automatisierte Injektionseinrichtung zur Durchführung der Thermodilution mit einer thermisch isolierten, auswechelbaren Zylinder-/Kolbenanordnung, deren Kolben mittels eines Kurbelgetriebes verfahrbar ist. Das Injektat wird aus dem Flüssigkeitsspeicher durch Verfahren des Kolbens angesaugt und durchläuft dabei eine Wärmetauscherkassette, in welcher die Flüssigkeit mittels einer thermoelektrischen Temperiervorrichtung gekühlt wird. Die Injektion des Bolus erfolgt durch Auspressen der Messflüssigkeit aus der Zylinder-/Kolbenanordnung, wobei eine Ventileinrichtung in der bereits zuvor beschriebenen Weise den Zustrom in die sowie aus der Zylinder-/Kolbenanordnung steuert.

Diese Vorrichtung weist den Nachteil eines erheblichen apparativen Aufwands zur Kühlung der Messflüssigkeit auf. Darüber hinaus kann nicht ausgeschlossen werden, dass sich das gekühlte Injektat während des relativ zeitintensiven Befüllens und Entleerens der Zylinder-/Kolbenanordnung trotz Isolierung in gewissem Maße erwärmt.

Aus der Patentschrift US 4,502,488 ist eine Injektionseinrichtung zur Durchführung einer Thermodilution mit ähnlichem konstruktivem Aufbau bekannt, welche mittels einer Anordnung mehrerer Ventile wahlweise aus einem ersten Speicher mit einer ersten Flüssigkeit oder aus einem zweiten, temperierten Speicher mit einer gekühlten Messflüssigkeit befüllt werden kann.

Weitere Vorrichtungen zur quasi-kontinuierlichen Durchführung der Thermodilution werden in den Patentschriften US 4,507,974 und US 6,736,782 offenbart, haben sich aber in der Praxis wegen des hohen Aufwands und aus Zuverlässigkeitserwägungen nicht durchgesetzt.

Die Patentschrift US 4,212,298 beschreibt einen Thermodilutions-Injektor mit einer Zylinder-/Kolbenanordung, welche zur Vergleichmäßigung des Injektionsvorgangs pneumatisch verfahrbar ist.

In den Patentschriften US 5,526,817 und US 4,327,724 werden außerdem Vorrichtungen zur manuellen oder teilautomatisierten Verabreichung des Flüssigkeitsbolus offenbart.

In der Patentschrift US 5,676,145 wird ein Katheter zur Durchführung der Thermodilution beschrieben, in welchen der Bolus der Messflüssigkeit mittels einer mit Kontroll- und Aufzeichnungsmitteln versehenen Infusionspumpe gepumpt wird. Der Katheter weist außerdem Mittel zur kontinuierlichen Bestimmung der Sauerstoffsättigung des Blutstroms auf.

Aus der Patentschrift US 6,004,275 ist ferner eine Vorrichtung zur Durchführung der Thermodilution zur Bestimmung des Herzzeitvolumens nach der Gorlin-Formel bekannt. Der in das Herz eingeführte Katheter ist dabei entlang seiner Erstreckung mit einer Anzahl von Öffnungen versehen, welche über im Katheter verlaufende, flüssigkeitsgefüllte Kanäle mit einer entsprechenden Anzahl von Druckmesseinrichtungen verbunden sind. Diese erfassen den Druck in der Arteria Pulmonaris, im rechten Vorhof sowie im linken Vorhof. Ferner weist der Katheter einen Temperaturfühler sowie einen aufweitbaren Ballon zur Verengung des Strömungsquerschnitts auf.

Aufgabe

Der vorliegenden Erfindung liegt einerseits die Aufgabe zugrunde, bei einer gattungsgemäßen Vorrichtung zur insbesondere wiederholt und automatisiert durchgeführten Bestimmung des Blutvolumens und/oder -volumenstroms eines Kreislaufabschnitts das Eindringen von Luft in das Injektat zuverlässig zu verhindern. Darüber hinaus soll die Vorrichtung dazu geeignet sein, Messungen mit therapeutisch notwendigen oder zumindest nicht kontraindizierten Flüssigkeitsmengen durchführen zu können. Weiterhin liegt der Erfindung die Aufgabe zugrunde, eine unerwünschte Beeinflussung des Messergebnisses durch eine Veränderung von Eigenschaften der Messflüssigkeit auf dem Wege zum Katheter weitmöglichst zu vermeiden. Ferner soll das Bedienpersonal rechtzeitig über die Durchführung der Messung informiert werden, um den Messvorgang nicht unnötig zu stören. Darüber hinaus soll das Messprogramm des Auswertegerätes fehlerhafte oder gestörte Dilutionsmessungen selbsttätig erkennen können und solche nicht als fehlerbehaftet anzeigen bzw. nicht zur Rekalibrierung einer anderen kontinuierlichen Methode zur Fluss- und/oder Volumenbestimmung verwenden.

Lösung

Die der Erfindung zugrunde liegende Aufgabe wird hinsichtlich der Bildung von Luftblasen bei einer gattungsgemäßen Vorrichtung dadurch gelöst, dass die Messflüssigkeit aus dem Flüssigkeitsspeicher mittels einer ersten Pumpeinrichtung einer zweiten Pumpeinrichtung und aus dieser dem Blutkreislauf zuführbar ist.

Zur Lösung der Aufgabe ist ferner eine Vorrichtung der eingangs genannten Art geeignet, bei welcher die Messflüssigkeit mit einem von der ersten Pumpeinrichtung bereitgestellten ersten Volumenstrom dem Blutkreislauf zuführbar ist, dem ein von der zweiten Pumpeinrichtung bereitgestellter zweiter Volumenstrom temporär, vorzugsweise in periodischer Wiederkehr, überlagerbar ist.

Durch diese Ausbildungen kann der in den Blutkreislauf eingespeiste Injektatvolumenstrom moduliert werden. Dabei tritt in der Regel keine rückwirkende Einflussnahme von der Mess- und Auswertevorrichtung auf die den Volumenstrom modulierenden Mittel auf. Die Handhabung und die Sicherheitsfunktionen der ersten Pumpeinrichtung bleiben uneingeschränkt erhalten. Ferner ist sichergestellt, dass im gesamten Infusionssystem kontinuierlich überatmosphärischer Druck herrscht und daher keine Luft in das System gelangt, so dass auf einen Luftdetektor in der Regel verzichtet werden kann.

Mit Bezug auf die unerwünschte Beeinflussung des Messergebnisses erfolgt die Lösung der erfindungsgemäßen Aufgabe bei einer Vorrichtung der eingangs genannten Art, welche mit Mitteln zur Konditionierung der Messflüssigkeit auf dem Weg zwischen Flüssigkeitsspeicher und Injektionsmittel ausgestattet ist, dadurch, dass die Mittel zur Konditionierung auf mindestens eine Pumpeinrichtung wirken. Damit wird gewährleistet, dass die Messflüssigkeit noch unmittelbar vor der Injektion die für die Messung günstigsten Eigenschaften aufweist.

Die Information des Bedienpersonals über die bevorstehende Messung erfolgt erfindungsgemäß dadurch, dass der Volumenstrom der ersten Pumpeinrichtung bestimmt und das Füllvolumen der zweiten Pumpeinrichtung festgelegt werden, daraus der Zeitpunkt berechnet wird, zu welchem die zweite Pumpeinrichtung befüllt ist und in zeitlichem Abstand vor der Aktivierung der zweiten Pumpeinrichtung ein Signal ausgegeben wird.

Darüber hinaus ist nach einer Ausbildung der Erfindung vorgesehen, dass die Vorrichtung mit einem Zulauf für körpereigenes Blut ausgestattet ist. Dem Körper des Patienten wird somit Blut entnommen, das nachfolgend als Messflüssigkeit eingesetzt wird und zu diesem Zweck mit zu Messzwecken geeigneten Eigenschaften versehen wird. Dabei kann Zulauf und Injektionsmittel eine Einrichtung zur Blutbehandlung, insbesondere zur Dialyse, angeordnet sein.

Die zu bestimmende Eigenschaft ist nach einer bevorzugten Ausbildung der Erfindung die Temperatur der Messflüssigkeit und im Blutkreislauf, da die Thermodilution als Verfahren zur Bestimmung des Blutvolumens und Blutvolumenstroms, insbesondere des Herzens, etabliert ist und sich die temporäre Abkühlung des Blutes ohne weiteres Zutun zurückstellt.

Die erste Pumpeinrichtung ist bevorzugt zur kontinuierlichen Förderung von Flüssigkeit geeignet und stellt einen zur zeitgerechten Befüllung der zweiten Pumpeinrichtung und/oder zur länger dauernden Injektion mit geringer Förderleistung ausreichenden Volumenstrom bereit. Aufgrund ihres mechanischen Aufbaus und der Sicherheitseinrichtungen besonders geeignet ist hierzu eine Schlauchpumpe, insbesondere eine übliche Infusionspumpe. Alternativ ist beispielsweise auch eine Pumpe einer arterio-venösen Hämodialysemaschine einzusetzen, wobei als Messflüssigkeit laufend zirkulierendes Eigenblut des Patienten verwendet wird.

Die zweite, unterstützende Pumpeinrichtung ist vorzugsweise zur diskontinuierlichen Förderung von Flüssigkeiten vorgesehen und wird zur Modulation der Injektatstroms alternativ oder zusätzlich zur ersten Pumpeinrichtung mit dem Katheter strömungsverbunden. Die zweite Pumpeinrichtung ist wegen ihres einfachen Aufbaus insbesondere eine Zylinder-/Kolbenanordnung. Alternativ kann auch eine Membranpumpe oder eine Plattenkompressionvorrichtung mit dazwischen befindlichem Injektatbeutel als zweite Pumpeinrichtung verwendet werden.

Nach einer ersten bevorzugten Alternative ist der Kolben der zweiten Pumpeinrichtung mit einer Antriebseinrichtung wirkverbindbar, die diesen zur Abgabe eines konstanten Volumenstroms gleichförmig in den Zylinder einschiebt.

Die Antriebseinrichtung ist dabei vorzugsweise ein pneumatische Antrieb, welcher nur auf den einzuschiebenden, nicht jedoch auf den ausfahrenden Kolben wirkt. Das Ausfahren des Kolbens wird dabei durch den Förderdruck der ersten Pumpeinrichtung bewirkt.

Mit besonderem Vorteil wirkt der Kolben in einer eingeschobenen und ausgefahrenen Endstellung auf Anschläge, welche über eine Steuerungseinrichtung, beispielsweise ein bistabiles 3/2-Wege-Ventil, die Antriebseinrichtung steuern.

Dabei sind zwischen der zweiten Pumpeinrichtung und der ersten Pumpeinrichtung ein Rückschlagventil und/oder stromab der zweiten Pumpeinrichtung mit Vorteil ein druckabhängig öffnendes Schwellventil angeordnet. Das Rückschlagventil verhindert bei Aktivierung der zweiten Pumpeinrichtung ein Rückströmen der Messflüssigkeit, während das Schwellventil beim Befüllen der zweiten Pumpeinrichtung den Durchfluss in Richtung des Katheters unterbindet.

Nach einer anderen vorteilhaften Alternative ist die Antriebseinrichtung zur Verringerung des konstruktiven Aufwands ein mit dem Kolben wirkverbundener Kraftspeicher, insbesondere eine Druckfeder. Dabei ist mit Vorteil vorgesehen, dass stromab der zweiten Pumpeinrichtung ein bistabiles Zwei-Wege-Ventil mit einer Sperrstellung und einer Durchgangsstellung angeordnet ist, welches beim Befüllen der Zylinder-/Kolbenanordnung die Strömungsverbindung zum Katheter sperrt und damit den Aufbau eines zum Spannen des Kraftspeichers erforderlichen Flüssigkeitsdrucks ermöglicht. Der Kolben wirkt vorzugsweise in einer eingeschobenen und ausgefahrenen Endstellung auf Anschläge, welche über eine Steuerungseinrichtung das Zwei-Wege-Ventil steuern.

Die Mittel zur Konditionierung der Messflüssigkeit in einer insbesondere der als Zylinder-/Kolbenanordnung ausgebildeten zweiten Pumpeinrichtung sind, da die Thermodilution das bevorzugte Messverfahren ist, vorzugsweise als Mittel zur Temperierung der Messflüssigkeit ausgebildet. Hierzu werden aufgrund ihrer Verfügbarkeit und der geringen Beschaffungskosten mit Vorteil handelsübliche Peltierelemente eingesetzt.

Die Vorrichtungen sind ferner mit Vorteil so ausgeführt, dass das Messflüssigkeit führende erfindungsgemäße Infusions-, Injektions- und Schlauchsystem, also zumindest Katheter und Schlauchleitung einschließlich von Verzweigungen abgehenden Strängen, als einmal verwendbares Sterilsystem aus verträglichen Materialien, insbesondere Polyurethan oder Polyaethylen oder Polycarbonat ausgebildet ist.

Figuren

In den Figuren sind beispielhaft und schematisch verschiedene Ausführungen der Erfindung dargestellt. Die angegebenen Parameter beziehen sich dabei auf einen normalgewichtigen Erwachsenen (Körpergewicht 70 kg).

Es zeigen:

Fig. 1: eine erste erfindungsgemäße Vorrichtung zur Bestimmung des Blutvolumens oder -volumenstroms eines Kreislaufabschnitts in Ausgangsstellung;

Fig. 2: die gleiche Vorrichtung nach Befüllen der zweiten Pumpvorrichtung;

Fig. 3: die Vorrichtung nach Fig. 1 und 2 nach Entleerung der zweiten Pumpvorrichtung;

Fig. 4: eine andere erfindungsgemäße Vorrichtung;

Fig. 5: eine Vorrichtung nach einer weiteren Ausbildung der Erfindung in Ausgangsstellung;

Fig. 6: dieselbe Vorrichtung nach Befüllen der zweiten Pumpvorrichtung;

Fig. 7: die Vorrichtung nach Fig. 5 und 6 nach Entleerung der zweiten Pumpvorrichtung;

Fig. 8: eine Vorrichtung nach einer anderen Ausführung.

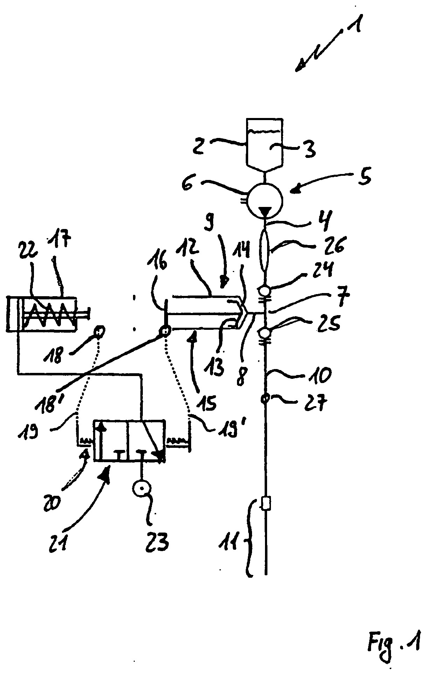

Die in Figur 1 dargestellte Vorrichtung 1 zur Bestimmung des Blutvolumens oder -volumenstroms eines Kreislaufabschnitts weist einen Flüssigkeitsspeicher 2 auf, beispielsweise einen Infusionsbeutel oder einen flaschenartigen Behälter, in welchem die Messflüssigkeit 3 bevorratet wird. Eine solche Messflüssigkeit kann beispielsweise eine Indikatorflüssigkeit sein. Die Messflüssigkeit 3 kann bereits im Flüssigkeitsspeicher 2 mit Eigenschaften ausgestattet werden, welche die Grundlage für den späteren Messvorgang bilden. Im Ausführungsbeispiel wird die Messflüssigkeit 3 für eine Thermodilution bereits in gekühltem Zustand, also mit einer Temperatur unterhalb der Körpertemperatur, bereitgestellt.

Das gesamte Messflüssigkeit 3 führende erfindungsgemäße Infusions-, Injektions- und Schlauchsystem ist bevorzugt als einmal verwendbares Sterilsystem aus verträglichen Materialien, beispielsweise Polyurethan oder Polyaethylen gefertigt.

Die Messflüssigkeit 3 wird über eine Schlauchleitung 4 mittels einer ersten Pumpeinrichtung 5 in Form einer Infusionspumpe 6 aus dem Flüssigkeitsspeicher 2 abgezogen und in Richtung einer Verzweigung 7 in der Schlauchleitung 4 gefördert, von der ein erster abzweigender Strang 8 mit einer zweiten Pumpeinrichtung 9 und der zweite abzweigende Strang 10 mit dem in den Blutkreislauf des Patienten eingeführten Katheter 11 verbunden ist.

Die zweite Pumpeinrichtung 9 ist spritzenartig ausgebildet und besteht aus einem Zylinder 12, in welchem ein Kolben 13 verschieblich angeordnet ist. Der Strang 8 mündet stirnseitig in die durch Verschieben des Kolbens 13 volumenveränderbare Kammer 14 der Zylinder-/Kolbenanordnung 15 ein. Das auskragende Ende 16 des Kolbens 13 trifft in seiner ausgefahrenen Endstellung auf eine in Initialstellung befindliche pneumatische Antriebseinrichtung 17 sowie auf einen Anschlag 18, welcher über eine mechanische Verbindung 19 mit der Verstelleinrichtung 20 eines pneumatischen 3/2-Wege-Ventils 21 wirkverbunden ist. In der eingefahrenen Endstellung des Kolbens 13 wird ein weiterer Anschlag 18' betätigt, der über eine mechanische Verbindung 19' gleichfalls mit der bistabilen Verstelleinrichtung 20 des 3/2-Wege-Ventils 21 verbunden ist. Anstelle der Anschläge 18, 18' und mechanischen Verbindungen 19, 19' können grundsätzlich auch elektrische oder pneumatische Schalter beziehungsweise Leitungen verwendet werden. Die Initialstellung des pneumatischen Antriebs 17 stellt sich unter der Wirkung einer Feder 22 selbständig ein, sobald das 3/2-Wege-Ventil 21 in seiner in Fig. 1 gezeigten Entlüftungsstellung den Antrieb 17 von dem Druckluftanschluss 23 trennt. Der Druckluftanschluss 23 kann beispielsweise mit dem Druckluftnetz eines Krankenhauses oder einem gesonderten Drucklufterzeuger verbunden sein.

Stromauf der Verzweigung 7 ist ein Rückschlagventil 24 in der Schlauchleitung 4 angeordnet, welches einen dem Förderstrom der ersten Pumpeinrichtung 5 entgegen gerichteten Rückfluss sperrt. Stromab der Verzweigung 7 befindet sich im Strang 10 ferner ein druckabhängig öffnendes Schwellventil 25.

Sofern es sich um eine Wiederholungsmessung handelt, befindet sich der Kolben 12 der Zylinder-/Kolbenanordnung 15 zu Beginn der Vorbereitung des Injektionsvorgangs in seiner in Fig. 1 gezeigten, maximal eingefahrenen Stellung, bei welcher sich das kleinstmögliche Volumen der Kammer 14 einstellt. Die Infusionspumpe 6 wird auf einen relativ geringen, aber konstanten Volumenstrom $V_1$ eingestellt, der beispielsweise zwischen 12,5 ml/h (=300 ml/24h) und 50 ml/h (=1.200 ml/24h)-betragen kann. Im Ausführungsbeispiel ist die Fördermenge auf 25 ml/h eingestellt. Beim Starten der Infusionspumpe 6 wird die von ihr geförderte Messflüssigkeit 3 unter Öffnung des Rückschlagventils 24 durch den Strang 8 der Schlauchleitung 4 hindurch in die Kammer 14 der Zylinder-/Kolbenanordnung 15 eingespeist, da der aus der Verzweigung 7 in Richtung des Katheters 11 führende Strang 10 der Schlauchleitung 4 durch das Schwellventil 25 gesperrt ist. Die beim Verschieben des Kolbens 13 zu überwindenden Friktionskräfte sind dabei konstruktiv so niedrig vorzugeben, dass der sich stromauf des Schwellventils 25 einstellende Druck in der Messflüssigkeit 3 unterhalb des Öffnungsdrucks des Schwellventils 25 liegt.

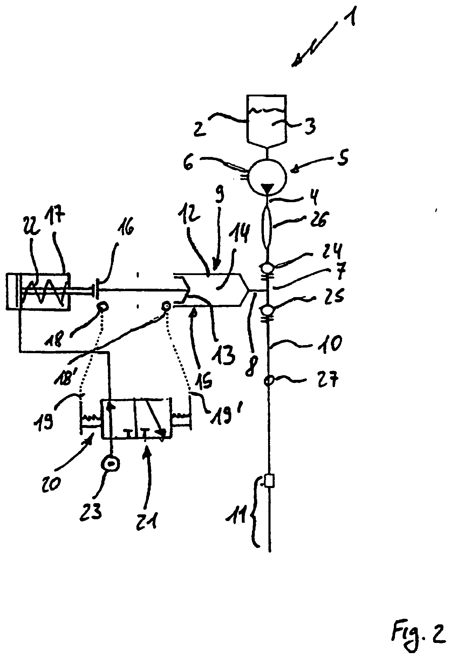

Beim Befüllen der im Ausführungsbeispiel maximal 25 ml fassenden Kammer 14 fährt der Kolben 13 aus dem Zylinder 12 heraus, bis sein auskragendes Ende 16 gegen den Anschlag 18 wirkt (Fig. 2). Das nun von der Kammer 14 aufgenommene Volumen an Messflüssigkeit 3 beträgt vorzugsweise zwischen 10 und 25 ml. Im Ausführungsbeispiel ist dieses mit 25 ml, also dem maximalen Fassungsvermögen der Kammer 14, eingestellt.

Die Vorrichtung 1 ist mit einem akustischen Warnsystem ausgestattet, welches mit einer Vorlaufzeit von beispielsweise 2 Minuten auf die bevorstehende Thermodilution hinweist. Maßnahmen, welche durch Beeinflussung der Bluttemperatur zu einer Verfälschung der Messergebnisse führen können, sind nachfolgend einzustellen.

Durch Betätigung des Anschlag 18 wird das 3/2-Wege-Ventil 21 mittels der bistabilen Verstelleinrichtung 20 in seine zweite Position verstellt, in welcher der Druckluftanschluss 23 mit dem pneumatischen Antrieb 17 verbunden ist und diesen gegen den ausgefahrenen Kolben 13 der zweiten Pumpeinrichtung 9 wirken lässt.

Der pneumatische Antrieb 17 ist so ausgelegt, dass die Füllung der Kammer 14 innerhalb von 1 bis 10 Sekunden mit einem konstanten und relativ hohen Volumenstrom $V_2$ aus der Zylinder-/Kolbenanordnung 15 ausgestoßen wird. Unter der Wirkung des sich dabei aufbauenden Förderdrucks schließt einerseits das Rückschlagventil 24, so dass ein Rückstrom in Richtung des Flüssigkeitsvorrats 2 unterbunden wird. Ein elastisch aufweitbarer Bereich 26 in der Schlauchleitung 4 nimmt die weiterhin von der Injektionspumpe 6 beförderte Messflüssigkeit stromauf des Rückschlagventils 24 auf. Die Elastizität des Bereichs 26 ist so zu wählen, dass der Druck in der Schlauchleitung 4 zwischen Rückschlagventil 24 und erster Pumpeinrichtung 5 sicher unter dem Abschaltdruck der Infusionspumpe 6 liegt.

Zeitgleich öffnet das Schwellventil 25 und gibt den Strömungskanal zwischen der zweiten Pumpeinrichtung 9 und dem Katheter 11 frei. Der über den Katheter 11 injizierte Volumenstrom $V_3$ entspricht dabei dem von der zweiten Pumpeinrichtung 9 eingespeisten Volumenstrom $V_2$. Das Schwellventil 25 ist vorzugsweise mit einer Einrichtung zur Messung des zentralvenösen Blutdrucks ausgestattet. Nach dem Öffnen des Schwellventils 25 tritt eine messbare Druckspitze bzw. eine länger dauernde Erhöhung des Druckes auf, deren Anstiegsflanke den Beginn des Injektionsvorgangs kennzeichnet und zur Aktivierung der Thermodilutions-Auswerteeinrichtung genutzt werden kann. Darüber hinaus wird im Strang 10 der Schlauchleitung 4 mittels eines Temperatursensors 27 die Temperatur der in den Katheter 11 fließenden Messflüssigkeit 3 erfasst. Der Verlauf der Bluttemperatur wird über einen Zeitraum von 10 bis 30 Sekunden gespeichert.

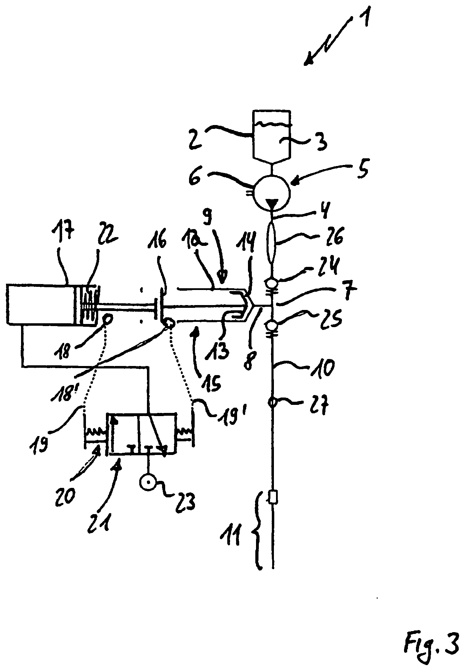

Ist das gewünschte Injektatvolumen aus der Zylinder-/Kolbenanordnung 15 ausgestoßen, trifft das auskragende Ende 16 des einfahrenden Kolbens 13 auf den zweiten Anschlag 18' mit der Folge, dass das 3/2-Wege-Ventil 21 den pneumatischen Antrieb 17 wieder vom Druckluftanschluss 23 trennt und entlüftet (Figur 3). Der pneumatische Antrieb 17 fährt nachfolgend unter der Wirkung der Feder 22 wieder in seine Ausgangsstellung nach Fig. 1 zurück. Gleichzeitig sinkt der Druck im Bereich der Verzweigung 7 ab, wodurch das Schwellventil 25 wieder schließt. Der Druckabfall charakterisiert ferner das Ende des Messvorgangs, so dass die für die Auswertung notwendige Dauer der Messung bestimmt werden kann. Der Förderdruck der Infusionspumpe 6 reicht nachfolgend aus, das Rückschlagventil 24 zu öffnen. Unter Entleerung des elastischen Bereichs 26 der Schlauchleitung erfolgt nun ein erneutes Befüllen der Zylinder-/Kolbenanordnung 15.

Berechnung und grafische Darstellung der Messergebnisse erfolgen durch die nicht dargestellte Auswerteeinheit automatisch. Mit dem über den Druckabfall im System bestimmbaren Ende der Messung erfolgt darüber hinaus eine Mitteilung, dass die Restriktionen hinsichtlich der Beeinflussung der Bluttemperatur bis zur Vorlaufzeit für die nächste Messung aufgehoben sind. Messungen werden verworfen, wenn beispielsweise die Bluttemperatur-Basislinie vor der Injektion über einen vorgegebenen Schwellenwert, beispielsweise +/- 0,1°/Minute, drifte oder das Signal/Rauschverhältnis (S/N) < 10 ist. Ferner sollte die Thermodilutionskurve bestimmte Kriterien aufweisen. So sollte beispielsweise der Anstieg steiler als der Abfall ausgebildet sein. Ferner sollte sich der Abfall im Wesentlichen monoexponentiell darstellen. Störungen oder Spitzen im Kurvenverlauf können gleichfalls auf eine ungültige Messung hinweisen.

Bei Kenntnis des von der Infusionspumpe 6 geförderten Volumenstroms $V_1$ und des Fassungsvermögens der Zylinder-/Kolbenanordnung 15 lassen sich die Zykluszeiten für die aufeinander abfolgenden Messungen berechnen. Üblicherweise ist die Zylinder-/Kolbenanordnung 15 vor Auslösung der ersten Messung bereits teilbefüllt, beispielsweise mit einem Volumen von 24 ml. Mit Inbetriebnahme der Vorrichtung 1 ist zunächst das zur vollständigen Füllung der Kammer 14 fehlende Volumen von 1 ml in die Zylinder-/Kolbenanordnung 15 einzuspeisen. Bei dem an der Infusionspumpe 6 eingestellten Volumenstrom $V_1$ von 25 ml/h erfolgt dies innerhalb von 2,4 Minuten (= 2 min, 24 sec). Unter Berücksichtigung der Fördertoleranzen der Infusionspumpe 6 von +/- 5% ergibt sich ein Zeitfenster von 2,28 Minuten (= 2 min 16,8 sec) bis 2,52 Minuten (= 2 min 31,2 sec). Darüber hinaus ist die Elastizität des Schlauchleitungssystems zu berücksichtigen, da infolge elastischer Aufweitung ein Teil der von der Infusionspumpe 6 geförderten Messflüssigkeit von der Schlauchleitung 4 aufgenommen wird. Dieses Totvolumen ist gerätespezifisch und kann damit vorab bestimmt und bei der Berechnung als Systemkonstante berücksichtigt werden. Falls das Totvolumen im Ausführungsbeispiel 1 ml beträgt, führt dies zu einer Verdoppelung der zur Vorbereitung der ersten Messung erforderlichen Zeit, also zwischen 4,56 Minuten und 5,04 Minuten. Für die zweite und nachfolgenden Messungen ist die Kammer 14 jeweils vollständig, also mit 25 ml zu befüllen. Unter Berücksichtigung des Totvolumens der Schlauchleitung 4 ergibt sich also eine Zykluszeit zwischen 57 Minuten und 63 Minuten. Die somit berechenbaren Zykluszeiten können zur Ausgabe der zuvor beschriebenen Hinweise auf bevorstehende Messungen benutzt werden.

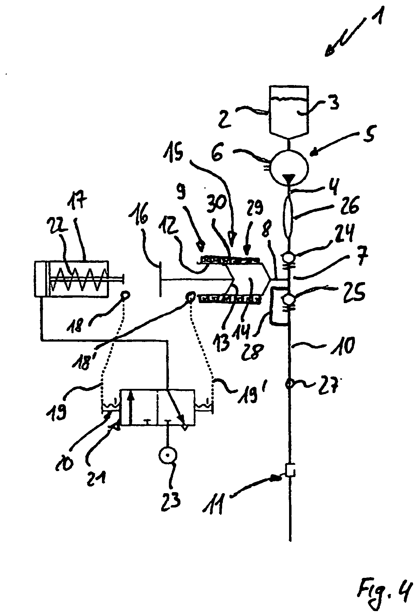

Das in Figur 4 gezeigte Ausführungsbeispiel entspricht im Wesentlichen der Vorrichtung nach Fig. 1 bis 3. Dem Schwellventil 25 ist jedoch eine als Bypass 28 wirkende Kapillare mit definiertem Flüssigkeitsdurchfluss zugeordnet, über welche auch während des Befüllens der zweiten Pumpenrichtung 9 ein Teilstrom $V_1$, des von der ersten Pumpeinrichtung 5 geförderten Volumenstroms $V_1$ zum Katheter 11 fließt. Während sich im ersten Ausführungsbeispiel ein Zyklus mit einer Phase hohen Injektionsdurchflusses und einer Phase ohne Injektion ausbildet, kann durch Verwendung des Bypasses 28 ein Alternieren von hohem und verringertem Durchfluss erzielt werden.

Die Zylinder-/Kolbenanordnung 15 ist ferner mit Mitteln 29 zur Temperierung der Messflüssigkeit 3 ausgestattet, durch welche eine Konditionierung während des Verweilens in der zweiten Pumpeinrichtung 9 hervorgerufen wird. Die Mittel 29 bestehen aus Peltierelementen 30, welche die äußere Mantelfläche des Zylinders 12 umgeben und nach dem Anlegen einer elektrischen Spannung kühlen.

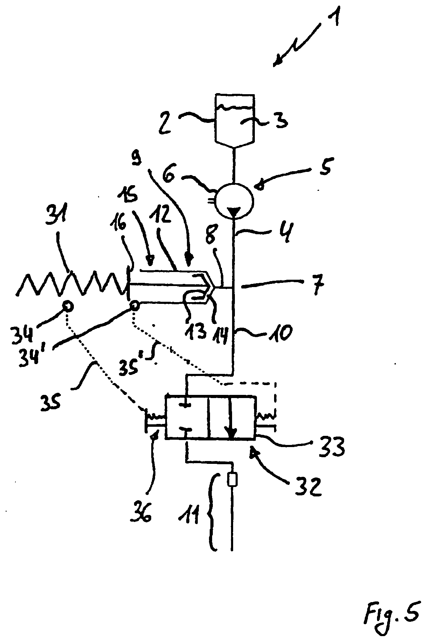

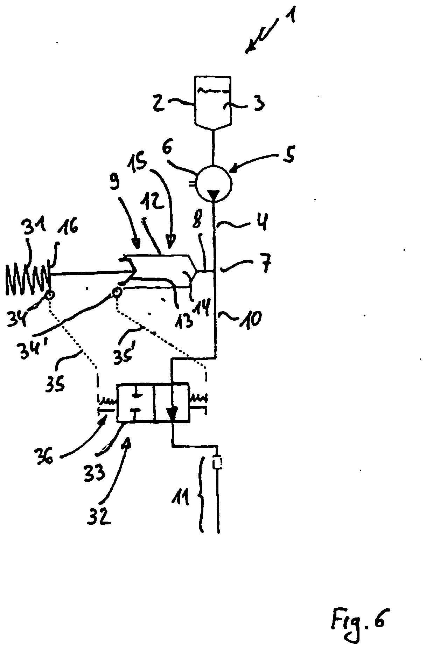

Bei der in den Figuren 5 bis 7 gezeigten Vorrichtung 1 wirkt der Kolben 13 der Zylinder-/Kolbenanordnung 15 unmittelbar gegen eine schraubenförmige Druckfeder 31, welche durch den bei Einspeisung von Messflüssigkeit 3 in die Kammer 14 ausfahrendem Kolben 13 gespannt wird. In der Schlauchleitung 4 ist stromab der Verzweigung 7 im Strang 10 eine Ventilanordnung 32 in Form eines Zwei-Wege-Ventils 33 angeordnet, welches eine Sperrstellung und eine Durchgangsstellung aufweist. Bei der Vorbereitung des Messvorgangs wird das Zwei-Wege-Ventil 33 in Sperrstellung gebracht (Fig. 5). Der von der Infusionspumpe 6 geförderte Volumenstrom $V_1$ wird daher aus der Verzweigung 7 über den Strang 8 in die Kammer 14 der Zylinder-/Kolbenanordnung 15 umgeleitet, welche sich hierdurch füllt. Der dabei heraus geschobene Kolben 13 wirkt bei Erreichen der gewünschten Füllmenge auf einen Anschlag 34, welcher über eine mechanische Verbindung 35 mit der Verstelleinrichtung 36 des mechanisch betätigten, bistabilen Zwei-Wege-Ventils 33 verbunden ist. Das Zwei-Weg-Ventil 33 wird hierdurch in die Durchgangsstellung verstellt, wodurch der Strömungskanal zwischen der Verzweigung 7 und dem Katheter 11 freigegeben wird (Fig. 6).

Durch diese Verstellung der Ventileinrichtung 32 kann der von der weiterhin tätigen Infusionspumpe 6 geförderte, konstante Volumenstrom $V_1$ an Messflüssigkeit 3 durch das Ventil 23 in den Katheter 11 fließen. Diesem wird ein zweiter Volumenstrom $V_2(t)$ überlagert, welcher von der Zylinder-/Kolbenanordnung 15 über den Strang 8 in die Schlauchleitung 4 zurückgespeist wird, wobei die Druckfeder 31 den Kolben 13 unter Verkleinerung des Volumens der Kammer 14 in den Zylinder 12 treibt. Der zweite Volumenstrom $V_2(t)$ nimmt mit der Zeit geringfügig ab, da die von der Druckfeder 31 erzeugte Druckkraft während des Einfahrens des Kolbens 13 infolge des Abbaus der Federspannung etwas abnimmt. Über einen Zeitraum von 1 bis 10 Sekunden tritt somit zunächst eine Phase ein, in welchem ein hoher, aber mit der Zeit abnehmender Volumenstrom $V_3(t) = V_2(t) + V_1$ über den Katheter 11 in den Blutkreislauf des Patienten injiziert wird. Durch eine geeignet dimensionierte Druckfeder 31 wird bevorzugt ein um maximal 20% vom rechteckigen Strömungsverlauf abweichender Volumenstrom $V_3(t)$ gewählt. Die Menge des von der Zylinder-/Kolbenanordnung 15 eingespeisten Menge an Messflüssigkeit 3 ist dabei, dem Fassungsvermögen der Kammer 14 zuzüglich der während der Injektionszeit von der Infusionspumpe gelieferten Messflüssigkeit entsprechend, bekannt.

Der Förderhöchstdruck der ersten Pumpeinrichtung 5 ist dabei so bemessen, dass er über dem höchsten Aktivierungsdruck der zweiten Pumpeinrichtung 9 liegt. Prinzip bedingt kann die zweiten Pumpeinrichtung 9 keinen höheren Druck als die erste Pumpeinrichtung 5 liefern. Damit kann keine Messflüssigkeit 3 aus der Zylinder-/Kolbenanordnung 15 entgegen der Förderrichtung der Infusionspumpe 6 in den Flüssigkeitsspeicher 2 zurückströmen. Der Förderdruck der Infusionspumpe 6 muss ausreichen, um den Kolben 13 der Zylinder-/Kolbeneinheit 15 gegen die Kraft der Druckfeder 31 auszufahren.

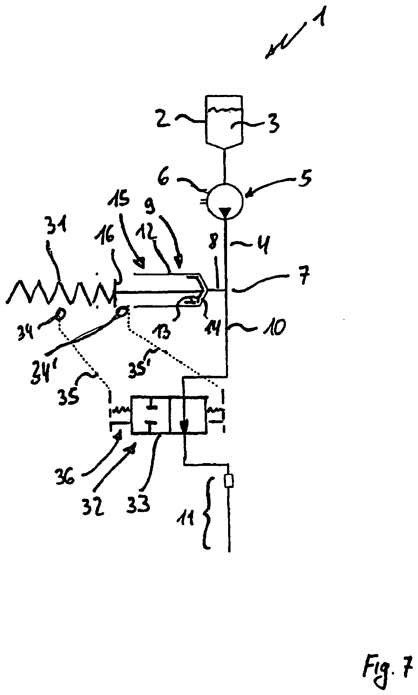

Nach der Entleerung der Kammer 14 (Figur 7) wirkt der in den Zylinder 12 eingeschobene Kolben 13 nun gegen einen weiteren Anschlag 34'. Dieser aktiviert die Verstelleinrichtung 36 des Zwei-Wege-Ventils 33, welches die Ventileinrichtung 32 in die Initialstellung nach Fig. 5 zurückstellt.

Anstelle der Anschläge 34, 34' und mechanischen Verbindungen 35, 35' können grundsätzlich auch elektrische oder pneumatische Schalter beziehungsweise Leitungen verwendet werden.

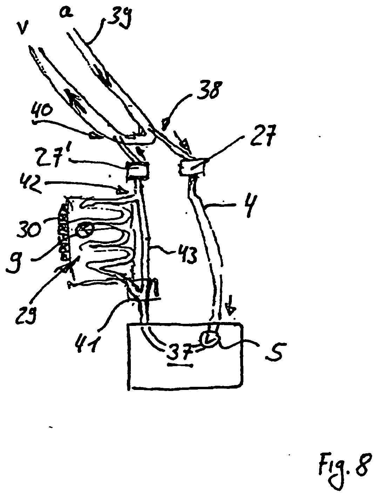

Bei der in Fig. 8 gezeigten Ausführung ist die Vorrichtung mit einer Einrichtung zur Blutbehandlung in Form einer Dialysemaschine 37 wirkverbunden, welche mit einer Pumpeinrichtung 5 zur im Wesentlichen kontinuierlichen Förderung eines Blutstroms ausgestattet ist. Die Dialysemaschine 37 ist über einen Zulauf 38 mit dem Gefäßsystem 39 des Dialysepatienten strömungsverbunden. Im Bereich des Zulaufs 38 ist im Schlauchsystem 4 ein erster Temperatursensor 27 angeordnet, welcher die Temperatur des aus dem Blutkreislauf entnommenen arteriellen Blutes misst. Ein weiterer Temperatursensor 27' befindet sich unmittelbar stromauf der venösen Shunt-Kanüle 40. Zwischen der Dialysemaschine 37 und dem Temperatursensor 27' ist ferner ein Mittel 29 zur Temperierung des dem Blutkreislauf entnommenen Blutes vorgesehen, dass dort mittels z.B. Peltierelementen 30 gekühlt wird. Das Mittel 29 zur Temperierung ist über ein aktiv gesteuertes Y-Ventil 41 und eine stromab davon befindliche Einmündung 42 mit der Schlauchleitung 4 strömungsverbunden, welche zwischen Y-Ventil 41 und Einmündung 42 einen Bypass 43 ausbildet.

Zur Vorbereitung der Messung wird durch Schalten des Y-Ventils 41 Blut in das Mittel 29 zur Temperierung in einer zur Durchführung der Thermodilution ausreichenden Menge eingespeist, wobei der Bypass 43 gesperrt wird. Nachfolgend wird das Y-Ventil 41 zurückgeschaltet, so dass das aus der Dialysemaschine 37 austretende Blut über den Bypass 43 in das Gefäßsystem 39 zurückfließt. Nach einer ausreichenden Abkühlung des zuvor gespeicherten Blutes erfolgt ein erneutes Schalten des Y-Ventils 41. Nunmehr treibt der von der Pumpeinrichtung 5 geförderte Blutstrom das abgekühlte, als Messflüssigkeit 3 dienende Blut in Richtung der venösen Shunt-Kanüle 40, wobei es den Temperatursensor 27' passiert.

Im Bereich des Mittels 29 zur Temperierung kann eine zweite, unterstützende Pumpeinrichtung 9 angeordnet werden, welche die Injektion des gekühlten Blutes beschleunigt. In der Regel wird diese jedoch nicht erforderlich sein, da sich bei einem üblichen Dialysepumpenfluss von 300 ml/min und einem Reservoir gekühlten Blutes von 20 ml eine ausreichend kurze Injektionszeit von 4 Sekunden ergibt.

Das Dialyseschlauchsystem einschließlich der Temperatursensoren 27, 27', des Y-Ventils 41, des Bypasses 43 und des beutelartigen Mittels 29 mit einem mäanderförmigen Blutdurchgang ist bevorzugt als Einwegartikel aus verträglichen Werkstoffen, beispielsweise Polyurethan, Polyethylen oder Polycarbonat, gefertigt.

In den Ausführungsbeispielen erfolgt die Messung des Blutvolumens oder -volumenstroms durch Verfolgung des zeitlichen Verlaufs der Bluttemperatur nach dem Injizieren eines gekühlten Flüssigkeitsbolus. Aussagekräftige Dilutionskurven können grundsätzlich jedoch auch durch Bestimmung der Auswirkungen anderer Eigenschaften von Messflüssigkeiten erfolgen, beispielsweise durch gezielte Veränderung der Sauerstoffsättigung im Blutstrom oder das Injizieren von Salzen, Farbstoffen oder radioaktiv markierten Substanzen. Die Themodilution ist jedoch das übliche Verfahren, da die durch den Injektatbolus hervorgerufene Abkühlung des Blutstroms zeitlich begrenzt ist und sich ohne weiteres Zutun zurückstellt.

Ferner kann beispielsweise durch Einfügen eines ansteuerbaren Sperr- oder Durchflussregelventils in den Strang 8 anstelle des starren Zeitmodells für die Phase hohen Durchflusses oder einer veränderbaren Verzögerung in der Verstelleinrichtung 20 oder 36 auch ein komplexes, zum Beispiel pseudo-zufälliges Muster erzeugt werden.

Bezugszeichenliste

| | |
|---|---|
| 1 | Vorrichtung (zur Bestimmung des Blutvolumens oder -volumenstroms eines Kreislaufabschnitts) |
| 2 | Flüssigkeitsspeicher |
| 3 | Messflüssigkeit |
| 4 | Schlauchleitung |
| 5 | Pumpeinrichtung |
| 6 | Infusionspumpe |
| 7 | Verzweigung |
| 8 | Strang |
| 9 | Pumpeinrichtung |
| 10 | Strang |
| 11 | Katheter |
| 12 | Zylinder |
| 13 | Kolben |
| 14 | Kammer |
| 15 | Zylinder-/Kolbenanordnung |
| 16 | Ende (des Kolbens) |

| 17 | Antrieb (pneumatisch) |
|---|---|
| 18, 18' | Anschlag |
| 19, 19' | Verbindung |
| 20 | Verstelleinrichtung |
| 21 | Zwei-Wege-Ventil |
| 22 | Feder |
| 23 | Druckluftanschluss |
| 24 | Rückschlagventil |
| 25 | Schwellventil |
| 26 | Bereich (elastisch) |
| 27, 27' | Temperatursensor |
| 28 | Bypass |
| 29 | Mittel (zur Temperierung) |
| 30 | Peltierelement |
| 31 | Druckfeder |
| 32 | Ventilanordnung |
| 33 | Zwei-Wege-Ventil |
| 34, 34' | Anschlag |
| 35, 35' | Verbindung |
| 36 | Verstelleinrichtung |
| 37 | Dialysemaschine |
| 38 | Zulauf |
| 39 | Gefäßsystem |
| 40 | Shunt-Kanüle |
| 41 | Y-Ventil |
| 42 | Einmündung |
| 43 | Bypass |
| $V_1$ | Volumenstrom (der ersten Pumpeinrichtung) |
| $V_2$ | Volumenstrom (der zweiten Pumpeinrichtung) |
| $V_3$ | Volumenstrom (im Katheter) |

**Patentansprüche**

1. Vorrichtung (1) zur Bestimmung des Blutvolumens und/oder Blutvolumenstroms eines Kreislaufabschnitts, mit

   - einem Flüssigkeitsspeicher (2) zur Bevorratung einer Messflüssigkeit (3),
   - einem Injektionsmittel, insbesondere einem Katheter (11) oder einer Kanüle, zur Injektion der Messflüssigkeit (3) in den Blutkreislauf,
   - mindestens einer zwischen dem Flüssigkeitsspeicher (2) und dem Injektionsmittel angeordneten ersten Pumpeinrichtung (5),
   - Mitteln zur Bestimmung mindestens einer Eigenschaft der Messflüssigkeit (3) vor der Injektion in den Blutkreislauf,
   - Mitteln zur Bestimmung der betreffenden Eigenschaft im Blutkreislauf stromab der Injektionsstelle und einer Auswerteeinrichtung zur Berechnung von Blutvolumen und/oder -volumenstrom in Abhängigkeit von den bestimmten Eigenschaften,

   **dadurch gekennzeichnet, dass**

die Messflüssigkeit (3) aus dem Flüssigkeitsspeicher (2) mittels der ersten Pumpeinrichtung (5) einer zweiten Pumpeinrichtung (9) und aus der zweiten Pumpeinrichtung (9) dem Blutkreislauf zuführbar ist.

2. Vorrichtung (1) nach Anspruch 1,
   **dadurch gekennzeichnet, dass**
   die Messflüssigkeit (3) mit einem von einer ersten Pumpeinrichtung (5) bereitgestellten ersten Volumenstrom (V1) dem Blutkreislauf zuführbar ist, dem ein von einer zweiten Pumpeinrichtung (9) bereitgestellter zweiter Volumenstrom (V2) temporär überlagerbar ist.

3. Vorrichtung nach Anspruch 2,
   **dadurch gekennzeichnet, dass**
   die temporäre Überlagerung periodisch wiederholbar ist.

4. Vorrichtung nach einem der Ansprüche 1 bis 3,
   **dadurch gekennzeichnet, dass**
   die zu bestimmende Eigenschaft die Temperatur der Messflüssigkeit (3) und im Blutkreislauf ist.

5. Vorrichtung nach einem der vorhergehenden Ansprüche,
   **dadurch gekennzeichnet, dass**
   die zweite Pumpeinrichtung (9) zur diskontinuierlichen Förderung der Messflüssigkeit (3) vorgesehen ist.

6. Vorrichtung nach Anspruch 5,
   **dadurch gekennzeichnet, dass**
   die zweite Pumpeinrichtung (9) eine Zylinder- /Kolbenanordnung (15) oder eine Membranpumpe ist.

7. Vorrichtung nach Anspruch 6,
   **dadurch gekennzeichnet, dass**
   der Kolben (13) der zweiten Pumpeinrichtung (9) mit einer Antriebseinrichtung wirkverbindbar ist.

8. Vorrichtung nach Anspruch 7,
   **dadurch gekennzeichnet, dass**
   zwischen der zweiten Pumpeinrichtung (9) und der ersten Pumpeinrichtung (5) ein Rückschlagventil (24) und/oder stromab der zweiten Pumpeinrichtung ein druckabhängig öffnendes Schwellventil (25) angeordnet sind.

9. Vorrichtung nach Anspruch 7,
   **dadurch gekennzeichnet, dass**
   die Antriebseinrichtung ein mit dem Kolben (13) wirkverbundener Kraftspeicher, insbesondere eine Druckfeder (31), ist.

10. Vorrichtung nach Anspruch 9,
    **dadurch gekennzeichnet, dass**
    stromab der zweiten Pumpeinrichtung (9) ein Zwei- Wege-Ventil (33) mit einer Sperrstellung und einer Durchgangsstellung angeordnet ist.

**11.** Vorrichtung nach Anspruch 9 oder 10,
**dadurch gekennzeichnet, dass**
der Kolben (13) in einer eingeschobenen und ausgefahrenen Endstellung auf Anschläge (34, 34') wirkt, welche über eine Steuerungseinrichtung das Zwei-Wege-Ventil (33) steuern.

**12.** Vorrichtung (1) nach einem der Ansprüche 1 bis 11
**dadurch, gekennzeichnet, dass**
die Vorrichtung mit Mitteln zur Konditionierung der Messflüssigkeit auf dem Weg zwischen Flüssigkeitsspeicher (2) und Injektionsmittel ausgestattet ist, wobei die Mittel zur Konditionierung auf mindestens eine Pumpeinrichtung (5, 9) wirken.

**13.** Vorrichtung nach Anspruch 12,
**dadurch gekennzeichnet, dass**
die Mittel zur Konditionierung als Mittel (29) zur Temperierung der Messflüssigkeit (3) ausgebildet sind.

**14.** Vorrichtung (1) gemäß einem der Ansprüche 1 bis 13, umfassend

- einer Schlauchleitung (4) zur Förderung einer Messflüssigkeit (3),

**dadurch gekennzeichnet, dass**
die Vorrichtung mit einem Zulauf für körpereigenes Blut ausgestattet ist.

**15.** Verfahren zum Betreiben der Vorrichtung nach einem der vorhergehenden Ansprüche,
**dadurch gekennzeichnet, dass**

- der Volumenstrom (V1) der ersten Pumpeinrichtung (5) bestimmt und das Füllvolumen der zweiten Pumpeinrichtung (9) festgelegt werden
- nachfolgend der Zeitpunkt berechnet wird, zu welchem die zweite Pumpeinrichtung (9) befüllt ist und
- in zeitlichem Abstand vor der Aktivierung der zweiten Pumpeinrichtung (9) ein Signal ausgegeben wird.

**Claims**

**1.** Apparatus (1) for determining the blood volume and/or blood volumetric flow of a circulation section, comprising

- a liquid reservoir (2) for storing a measuring liquid (3),
- an injecting means, in particular a catheter (11) or a cannula, for injecting the measuring liquid (3) into the blood circulation,
- at least one first pumping device (5) arranged between the liquid reservoir (2) and the injecting means,
- means for determining at least one characteristic of the measuring liquid (3) before injection into the blood circulation,
- means for determining the respective characteristic within the blood circulation downstream of the injection location, and an evaluation device for calculating the blood volume and/or blood volumetric flow as a function of the determined characteristics,

**characterized in that** the measuring liquid (3) can be supplied from the liquid reservoir (2) to a second pumping device (9) by means of the first pumping device (5) and from the second pumping device (9) to the blood circulation.

**2.** Apparatus (1) according to claim 1,
**characterized in that**
the measuring liquid (3) can be supplied to the blood circulation with a first volumetric flow (V1) provided by the first pumping device (5) and temporarily superposed by a second volumetric flow (V2) provided by the second pumping device (9).

**3.** Apparatus according to claim 2,
**characterized in that**
the temporary superposition is repeatable periodically.

**4.** Apparatus according to one of the claims 1 to 3,
**characterized in that**
the characteristic to be determined is the temperature of the measuring liquid (3) and within the blood circulation.

**5.** Apparatus according to one of the preceding claims,
**characterized in that**
the second pumping device (9) is provided for a discontinuous conveyance of the measuring liquid (3).

**6.** Apparatus according to claim 5,
**characterized in that**
the second pumping device (9) is a cylinder/piston assembly (15) or a diaphragm pump.

**7.** Apparatus according to claim 6,
**characterized in that**
the piston (13) of the second pumping device (9) is operatively connectable to a driving device.

**8.** Apparatus according to claim 7,
**characterized in that**
a check valve (24) is arranged between the second pumping device (9) and the first pumping device (5) and/or a pressure-dependently opening swell valve (25) is arranged downstream of the second pumping device (9).

9. Apparatus according to claim 7,
**characterized in that**
the driving device is a energy storage, in particular a compression spring (31), which is operatively connected to the piston (13).

10. Apparatus according to claim 9,
**characterized in that**
a two-way valve (33) having a blocking position and a passing position is arranged downstream of the second pumping device (9).

11. Apparatus according to claim 9 or 10,
**characterized in that**
the piston (13) in a retracted end position and in an extracted end position is acting on stops (34, 34') controlling the two-way valve (33) via a control device.

12. Apparatus (1) according to one of the claims 1 to 11,
**characterized in that**
the apparatus is provided with means for conditioning the measuring liquid on the way between the liquid reservoir (2) and the injecting means, wherein the means for conditioning are acting on at least one pumping device (5, 9).

13. Apparatus according to claim 12,
**characterized in that**
the means for conditioning are formed as means (29) for tempering the measuring liquid (3).

14. Apparatus (1) according to one of the claims 1 to 13, comprising

- a hose pipe (4) for conveying the measuring liquid (3),

**characterized in that**
the apparatus is provided with an inlet for the body's blood.

15. Method of operating an apparatus according to one of the preceding claims,
**characterized in that**

- the volumetric flow (V1) of the first pumping device (5) is determined and the filling volume of the second pumping device (9) is ascertained,
- subsequently, a point in time is calculated at which the second pumping device (9) is filled, and
- a signal is output with a time distance before activation of the second pumping device (9).

**Revendications**

1. Dispositif (1) destiné à déterminer le volume sanguin et/ou le débit sanguin dans une partie donnée de la circulation sanguine, comprenant

- un réservoir de liquide (2) destiné à stocker un liquide de mesure (3),
- un moyen d'injection, en particulier un cathéter (11) ou une canule, destiné à injecter le liquide de mesure (3) dans la circulation sanguine,
- au moins un premier dispositif de pompage (5) disposé entre le réservoir de liquide (2) et le moyen d'injection,
- des moyens destinés à déterminer au moins une propriété du liquide de mesure (3) avant son injection dans la circulation sanguine,
- des moyens destinés à déterminer ladite propriété dans la circulation sanguine en aval du point d'injection et un dispositif d'évaluation destiné à calculer un volume sanguin et/ou un débit sanguin en fonction des propriétés déterminées,

**caractérisé en ce que**
le liquide de mesure (3) peut être amené, au moyen du premier dispositif de pompage (5), du réservoir de liquide (2) à un deuxième dispositif de pompage (9) et introduit dans la circulation sanguine à partir du deuxième dispositif de pompage (9).

2. Dispositif (1) selon la revendication 1,
**caractérisé en ce que**
le liquide de mesure (3) peut être introduit dans la circulation sanguine avec un premier débit (V1) fourni par un premier dispositif de pompage (5) auquel peut se superposer de manière temporaire un deuxième débit (V2) fourni par un deuxième dispositif de pompage (9).

3. Dispositif selon la revendication 2,
**caractérisé en ce que**
la superposition temporaire peut se répéter de manière périodique.

4. Dispositif selon l'une quelconque des revendications 1 à 3,
**caractérisé en ce que**
la propriété à déterminer est la température du liquide de mesure (3) et celle au sein de la circulation sanguine.

5. Dispositif selon l'une quelconque des revendications précédentes,
**caractérisé en ce que**
le deuxième dispositif de pompage (9) est prévu pour refouler le liquide de mesure (3) de manière discontinue.

**6.** Dispositif selon la revendication 5, **caractérisé en ce que** le deuxième dispositif de pompage (9) est un système piston-cylindre (15) ou une pompe à membrane.

**7.** Dispositif selon la revendication 6, **caractérisé en ce que** le piston (13) du deuxième dispositif de pompage (9) peut coopérer avec un dispositif d'entraînement.

**8.** Dispositif selon la revendication 7, **caractérisé en ce que** un clapet anti-retour (24) est disposé entre le deuxième dispositif de pompage (9) et le premier dispositif de pompage (5) et/ou qu'un clapet à seuil (25) qui s'ouvre en fonction de la pression est disposé en aval dudit deuxième dispositif de pompage.

**9.** Dispositif selon la revendication 7, **caractérisé en ce que** le dispositif d'entraînement est un accumulateur d'énergie, en particulier un ressort de compression (31), qui coopère avec le piston (13).

**10.** Dispositif selon la revendication 9, **caractérisé en ce que** un clapet à deux voies (33) comprenant une position de blocage et une position de passage est disposé en aval du deuxième dispositif de pompage (9).

**11.** Dispositif selon la revendication 9 ou 10, **caractérisé en ce que** le piston (13) agit, lorsqu'il se trouve dans une position finale rentrée et dans une position finale sortie, sur des butées (34, 34') qui commandent le clapet à deux voies (33) par l'intermédiaire d'un dispositif de commande.

**12.** Dispositif (1) selon l'une quelconque des revendications 1 à 11, **caractérisé en ce que** ledit dispositif est équipé de moyens destinés à conditionner le liquide de mesure sur le trajet entre le réservoir de liquide (2) et le moyen d'injection, les moyens de conditionnement agissant sur au moins un dispositif de pompage (5, 9).

**13.** Dispositif selon la revendication 12, **caractérisé en ce que** les moyens de conditionnement sont conçus en tant que moyens (29) destinés à réguler la température du liquide de mesure (3).

**14.** Dispositif (1) selon l'une quelconque des revendications 1 à 13, comprenant

    - un conduit souple (4) destiné à transporter un liquide de mesure (3),

**caractérisé en ce que** ledit dispositif est équipé d'une entrée pour le sang natif.

**15.** Procédé destiné à faire fonctionner le dispositif selon l'une quelconque des revendications précédentes, **caractérisé en ce que**

    - le débit (V1) du premier dispositif de pompage (5) et le volume de remplissage du deuxième dispositif de pompage (9) sont fixés,
    - ensuite, l'instant du remplissage complet du deuxième dispositif de pompage (9) est calculé, et
    - un signal est émis à un certain intervalle de temps avant l'activation du deuxième dispositif de pompage (9).

Fig. 1

Fig. 2

Fig. 3

Fig. 4

Fig. 5

Fig. 6

Fig. 7

Fig. 8

20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4214068 A1
- US 4817624 A
- US 4507974 A
- US 5037396 A
- US 4502488 A
- US 6736782 B
- US 4212298 A
- US 5526817 A
- US 4327724 A
- US 5676145 A
- US 6004275 A